# EUROPEAN PATENT APPLICATION

(11) **EP 3 520 781 A1**
(43) Date of publication of application: **07.08.2019**
(21) Application number: 18461514.4
(22) Date of filing: 05.02.2018
(51) Int. Cl.: A61K 9/16, A61K 31/4152, A61K 31/472, A61K 31/522, A61K 9/24

(54) **A PHARMACEUTICAL COMPOSITION COMPRISING METAMIZOLE, DROTAVERINE, AND CAFFEINE**

(71) Applicant: Adamed sp. z o.o., 05-152 Czosnów k/Warszawy (PL)
(72) Inventor: KLECZAR, Katarzyna, 32-600 Oswiecim (PL); CIEPLUCHA, Agnieszka, 05-825 Opypy (PL)
(74) Representative: Sulikowski, Daniel

(57) **Abstract**

The present inventions relates to a pharmaceutical composition comprising, in one dosage form, metamizole, drotaverine, and caffeine, in which metamizole in the said composition is present in the form of a granulate comprising metamizole, and drotaverine and caffeine are present outside the said granulate. The present inventions relates also to a granulate comprising metamizole and a granulate comprising drotaverine, as well as their use.

## Description

The present invention relates to a pharmaceutical composition comprising metamizole, drotaverine, and caffeine. The present invention relates also to a granulate comprising drotaverine, and the use of the said granulate, as well as the use of a granulate comprising metamizole.

The combination of metamizole, drotaverine, and caffeine is known for its strong analgesic effect. Such a combination preferably can be used in the case of a severe and persistent pain and fever. In addition, the combination of an analgesic substance (metamizole) with an antispasmodic substance (drotaverine) effectively relieves spastic pains occurring *inter alia* during migraine, menstruation, colic, etc. The third active substance (caffeine) enables faster action of the medicine and intensifies the analgesic effects of the other substances (metamizole and drotaverine).

To the best of the Applicant's knowledge, only one product comprising metamizole, drotaverine, and caffeine, sold under the name of Quarelin or Algopyrin Complex, is available on the market. In addition to the above-mentioned active substances at doses of, respectively, 400 mg/40 mg/60 mg, a tablet consists of talc, magnesium stearate, polyvinylpyrrolidone, microcrystalline cellulose, and starch.

Unfortunately, this combination of the above-mentioned medicinal substances, which is the only one available on the market, fails to meet the requirements of chemical purity. The chemical quality deteriorates further over time during the storage of the medicine, as the amounts of impurities increase due to the degrading active components. Particular attention should be paid to the high level of impurities of drotaverine derivatives, the limit of which for a single unknown impurity is 0.2%. It is, therefore, desirable to provide a pharmaceutical composition comprising these three active substances with good stability.

Therefore, the present invention relates to a pharmaceutical composition comprising, in one dosage form, metamizole, drotaverine, and caffeine, characterised in that metamizole in the said composition is present in the form of a granulate containing metamizole, and drotaverine and caffeine are present outside the said granulate.

Whenever the present application mentions metamizole, drotaverine, or caffeine, each time it is also understood as relevant pharmaceutically acceptable salts of the above-mentioned active substances.

A "pharmaceutically acceptable salt" is a salt whose counter-ion that forms a salt with, respectively, metamizole, drotaverine or caffeine exhibits no toxic effect at normally used doses of these salts. Preferably, in this invention, metamizole is used in the form of a sodium salt (metamizole sodium), drotaverine is used in the form of drotaverine hydrochloride, while caffeine is used in the form of a free base.

The invention involves the physical separation of metamizole from the other active components of the composition, wherein metamizole has to be in the form of a granulate. The other two components, namely drotaverine and caffeine, have to be present outside the granulate comprising metamizole in order to ensure the stability of the pharmaceutical composition being manufactured.

In one embodiment, the granulate comprising metamizole is a wet granulate. The term "wet granulate" denotes that such a granulate has been prepared using the wet granulation technique known to those skilled in the art of pharmacy. Preferably, to obtain a wet granulate, either the wet granulation technique in a high-shear mixer or the fluid bed granulation technique is applied.

Alternatively, in another embodiment, the granulate comprising metamizole is a dry granulate. The term "dry granulate" denotes that such a granulate has been prepared using the dry granulation technique known to those skilled in the art of pharmacy. Preferably, to obtain a dry granulate, the roller-compaction or briquetting techniques are applied.

In a preferred embodiment, the above-mentioned granulate, both wet and dry, consists of:
metamizole or its pharmaceutical acceptable salt at an amount of 70 to 90% wt.,
at least one filler at an amount of 5 to 20% wt.,
at least one binder at an amount of 0 to 5% wt.,
at least one disintegrant at an amount of 0 to 5% wt.,
at least one lubricant at an amount of 0 to 1% wt.,
wherein the percentages are each time related to the total weight of the granulate, and each time sum up to 100%.

Preferably, drotaverine and caffeine are present together in one dry granulate comprising drotaverine and caffeine.

In a preferred embodiment, the granulate comprising drotaverine and caffeine consists of:
drotaverine or its pharmaceutically acceptable salt at an amount of 15 to 30% wt.,
caffeine at an amount of 25-35% wt.,
at least one filler at an amount of 30 to 50% wt.,
at least one binder at an amount of 0 to 5% wt.,
at least one disintegrant at an amount of 0 to 5% wt.,
at least one lubricant at an amount of 0 to 6% wt.,
wherein the percentages are each time related to the total weight of the granulate, and each time sum up to 100%.

Alternatively, preferably drotaverine is present in the form of a dry granulate comprising drotaverine, while caffeine is present in the form of a powder outside the said granulate comprising drotaverine. In such a case, caffeine is preferably added in the form of a powder, with no additional components in the form of at least one filler, binder, disintegrant, or lubricant, although in certain cases the addition of such agents can be preferable. A person skilled in the art will be able to adjust appropriate amounts and types of substances in the form of a powder.

In a preferred embodiment, the granulate comprising drotaverine consists of:
drotaverine or its pharmaceutical acceptable salt at an amount of 20 to 55% wt.,
at least one filler at an amount of 40 to 75% wt.,
at least one binder at an amount of 0 to 5% wt.,
at least one disintegrant at an amount of 0 to 5% wt.,
at least one lubricant at an amount of 0 to 6% wt.,
wherein the percentages are each time related to the total weight of the granulate, and each time sum up to 100%.

Alternatively, preferably drotaverine and caffeine are present in the form of a mixture of powders. In such a case, both drotaverine and caffeine are preferably added in the form of a powder, with no additional components in the form of at least one filler, binder, disintegrant, or lubricant, although in certain cases the addition of such agents can be preferable. A person skilled in the art will be able to adjust appropriate amounts and types of substances in the form of a powder.
In a preferred embodiment,
at least one filler is selected from a group consisting of lactose, microcrystalline cellulose, and maize starch,
at least one binder is selected from a group consisting of PVP, and HPMC,
at least one disintegrant is selected from a group consisting of crospovidone, and croscarmellose,
at least one lubricant is selected from a group consisting of talc and magnesium stearate.

Another subject-matter of the invention is a dry granulate comprising drotaverine, characterised in that it does not contain acid stabilisers. Unexpectedly, it turned out that the dry granulate comprising drotaverine exhibited high stability without the presence of additional agents in the form of acid stabilisers. Without wishing to be bound by theory, in the Applicant's opinion this is due to the elimination of water in the process through the application of dry granulation. Dry granulation may be particularly preferable in the case of heterogeneous or different API batches e.g. different particle size distribution, different bulk density, in order to obtain repeatable and appropriate quality of the intermediate and finished product, e.g. content uniformity, weight uniformity, flow.

The term "acid stabiliser" should be understood as an addition in the form of an organic acid with the dissociation constant (K) of the order of 10⁻³-10⁻¹² at a temperature of 25°C. An acid stabiliser preferably may be selected from a group consisting of citric acid, ascorbic acid.

Preferably, a dry granulate comprising drotaverine consists of:
drotaverine at an amount of 20 to 55% wt.,
at least one filler at an amount of 40 to 75% wt.,
at least one binder at an amount of 0 to 5% wt.,
at least one disintegrant at an amount of 0 to 5% wt.,
at least one lubricant at an amount of 0 to 6% wt.,

More preferably, the said granulate comprises
at least one filler is selected from a group consisting of lactose, microcrystalline cellulose, and maize starch,
at least one binder is selected from a group consisting of PVP, and HPMC,
at least one disintegrant is selected from a group consisting of crospovidone, and croscarmellose,
at least one lubricant is selected from a group consisting of talc and magnesium stearate.

Moreover, the present invention relates to the use of the dry granulate comprising drotaverine for the manufacturing of a pharmaceutical composition comprising drotaverine.

In addition, the present invention relates to also the use of the granulate comprising metamizole for the manufacturing of a pharmaceutical composition comprising metamizole, drotaverine, and caffeine.

### EXAMPLES

### 1. Description and composition of intermediates

### 1.1. Intermediates of metamizole

During the development, metamizole was processed into the form of either wet or dry granulate, in order to investigate the effect of the presence of water in the technological process on chemical purity of APIs and on further processability of the manufactured intermediate into the final form, namely a tablet. Below, examples of qualitative and quantitative compositions as well as a technological description of the tests carried out for Metamizole Sodium (in the monohydrate form) are provided. The aforementioned qualitative and quantitative compositions, appropriately processed using the wet and dry granulation technology, were aimed at checking on the type and amount of the binder (e.g. PVP, HPMC), filler (e.g. cellulose, lactose, starch), disintegrant (e.g. crospovidone, croscarmellose), and lubricant (e.g. magnesium stearate) on physical and chemical quality of the obtained granulate and the quality of the manufacturing process. Due to the high water content (approx. 5%) in the API, which corresponds to the fact that the substance is a monohydrate, the weighed amount of Metamizole Sodium is converted from molar mass (421.24 mg of Metamizole Sodium monohydrate is equivalent to 400.00 mg of Metamizole Sodium).

### 1.1.1. A wet granulate comprising metamizole, obtained using the wet granulation technology.

**Table 1. Examples of qualitative and quantitative compositions for wet granulates of MET.**

| Technology | Wet granulation (High-shear granulation) | | | | | | | |
|---|---|---|---|---|---|---|---|---|
| Components | Composition No [mg/tablet] | | | | | | | |
| | **MW1**. | **MW2**. | **MW3.** | **MW4.** | **MW5.** | **MW6.** | **MW7.** | **MW8.** |
| Metamizole Sodium Monohydrate | 421.24 | 421.24 | 421.24 | 421.24 | 421.24 | 421.24 | 421.24 | 421.24 |
| Cellulose, microcrystalline | 80.76 | 40.76 | 60.76 | 60.76 | 20.76 | 60.76 | 20.76 | 60.76 |
| Lactose monohydrate | - | 40.00 | 20.00 | - | 60.00 | 20.00 | - | - |
| Maize starch | - | - | - | 20.00 | | | 60.00 | 20.00 |
| Povidone | 20.00 | 20.00 | 20.00 | 20.00 | 25.00 | - | - | - |
| HMPC | - | - | - | - | - | 20.00 | 20.00 | 20.00 |
| Crospovidone | 20.00 | 25.00 | | | 25.00 | 25.00 | - | 25.00 |
| Croscarmellose | - | - | 25.00 | 25.00 | - | - | 25.00 | - |
| Total: | 542.00 | 547.00 | 547.00 | 547.00 | 552.00 | 547.00 | 547.00 | 547.00 |

The process of granulate manufacturing included the following steps:
- pre-calibration / unification of starting materials (granulation bed components, including the active substance),
- mixing of the aforementioned in a high-shear granulator to obtain a uniform distribution of the active substance within the granulation bed,
- preparation of an aqueous solution of the binder, and the application thereof onto the bed,
- proper granulation (manufacturing of the granulate) in a Glatt TMG1/6 high-shear granulator,
- drying of the granulate in order to remove water provided in the binder solution,
- calibration / unification of the dry granulate.

### 1.1.2. Wet granulate of MET, obtained using the dry granulation technology.

**Table 2. Examples of qualitative and quantitative compositions for dry granulates of MET.**

| Technology: | Dry granulation | | | | | | | |
|---|---|---|---|---|---|---|---|---|
| Components | Composition No [mg/tablet] | | | | | | | |
| | **MD1.** | **MD2.** | **MD3** | **MD4.** | **MD5.** | **MD6.** | **MD7.** | **MD8.** |
| Metamizole Sodium Monohydrate | 421.24 | 421.24 | 421.24 | 421.24 | 421.24 | 421.24 | 421.24 | 421.24 |
| Cellulose, microcrystalline | 61.76 | 31.76 | 51.76 | 51.76 | 56.76 | 86.76 | 111.76 | 80.76 |
| Lactose monohydrate | - | 31.00 | 11.00 | 11.00 | 54.00 | 24.00 | 49.00 | 75.00 |
| Crospovidone | 16.00 | - | - | - | - | - | 20.00 | - |
| Croscarmellose | - | 16.00 | 16.00 | 16.00 | 18.00 | 18.00 | - | 20.00 |
| Magnesium stearate | - | - | - | - | - | - | - | 5.00 |
| Povidone | - | - | - | 15.00 | - | - | - | - |
| Total: | 500.00 | 500.00 | 500.00 | 515.00 | 550.00 | 550.00 | 600.00 | 600.00 |

The process of granulate manufacturing included the following steps:
- pre-calibration / unification of starting materials (granulation components, including the APIs),
- mixing of the aforementioned components to obtain a uniform distribution of the active substance within the granulation bed (compositions 1-7). In the case of composition 8, in the first step, all components were mixed except the lubricant which was mixed in the second step,
- dry granulation, using an Alexanderwerk BT 120 Pharma compactor, of the aforementioned mixture at standard process parameters, in order to obtain briquettes which were subsequently processed into fine particles and unified.

### 1.2 Intermediate of drotaverine and caffeine

As intermediates of Drotaverine Hydrochloride and Caffeine, a dry granulate comprising Drotaverine as well as a common dry granulate of Drotaverine and Caffeine was prepared. In addition, in one embodiment, a possibility was checked for the addition of an unprocessed powder mixture of Drotaverine and a powder mixture of Caffeine, wherein such a mixture comprises, besides the active substance, a filler, e.g. microcrystalline cellulose or lactose or a mixture thereof, directly to the final mixture with the intermediate comprising metamizole.

The following qualitative and quantitative compositions were subjected to the process of dry granulation, and were aimed at checking on the effect of the type and amount of the filler (e.g. cellulose, lactose), disintegrant (e.g. crospovidone, croscarmellose), binder (e.g. PVP), and the lubricating and anti-adhesive substance (e.g. magnesium stearate, talc) on physical and chemical quality of the obtained granulate, and on the quality of the tableting process.

### 1.2.1. A dry granulate of Drotaverine and Caffeine, manufactured using the dry granulation technology.

**Table 3. Examples of qualitative and quantitative compositions for dry granulates of DRO+CAF.**

| Technology: | Dry granulation | | | | |
|---|---|---|---|---|---|
| Components | Composition No [mg/tablet] | | | | |
| | **DC1.** | **DC2.** | **DC3** | **DC4.** | **DC5.** |
| Drotaverine hydrochloride | 40.00 | 40.00 | 40.00 | 40.00 | 40.00 |
| Caffeine, anhydrous | 60.00 | 60.00 | 60.00 | 60.00 | 60.00 |
| Cellulose, microcrystalline | 37.00 | 37.00 | 52.00 | 73.00 | 73.00 |
| Lactose monohydrate | 37.00 | 37.00 | 22.00 | - | - |
| Crospovidone | - | 6.00 | - | - | - |
| Croscarmellose | 6.00 | - | 6.00 | 6.0 | 6.0 |
| Magnesium stearate | - | - | - | 1.0 | - |
| Talc | - | - | - | - | 10.00 |
| Povidone | - | 5.0 | - | - | - |
| Total: | 180.00 | 185.00 | 180.00 | 180.00 | 190.00 |

The process of granulate manufacturing included:
- Pre-calibration / unification of starting materials (granulation components, including the APIs),
- mixing of the aforementioned components to obtain a uniform distribution of the active substance within the granulation bed (compositions 1-3). In the case of compositions 4 and 5, in the first step, all components were mixed except the lubricating and anti-adhesive substances which were mixed in the second step,
- Dry granulation using an Alexanderwerk BT 120 Pharma compactor, of the aforementioned mixture at standard process parameters, in order to obtain briquettes which were subsequently processed into fine particles and unified

### 1.2.2. A dry granulate of Drotaverine, manufactured using the dry granulation technology.

A considerably lower dose of DRO (40 mg) in relation to metamizole enabled, to the greater extent, checking on the effect of the amount of auxiliary components on physical parameters of the obtained granulate and the chemical stability thereof. In the case of metamizole, it was not possible to obtain significant dilution of the API in the intermediate without a considerable effect on the final weight of the tablet.

**Table 4. Examples of qualitative and quantitative compositions for dry granulates of DRO.**

| Technology: | Dry granulation (Compacting) | | | | |
|---|---|---|---|---|---|
| Components | Composition No [mg/tablet] | | | | |
| | **D1.** | **D2.** | **D3** | **D4.** | **D5.** |
| Drotaverine hydrochloride | 40.00 | 40.00 | 40.00 | 40.00 | 40.00 |
| Cellulose, microcrystalline | 18.5 | 32.00 | - | 66.5 | 96.5 |
| Lactose monohydrate | 18.5 | - | 37.00 | 66.5 | 36.5 |
| Crospovidone | 3.00 | 3.00 | - | 7.00 | 7.00 |
| Croscarmellose | - | - | 3.00 | - | - |
| Talc | | 5.00 | - | - | - |
| Total: | 80.00 | 80.00 | 80.00 | 180.00 | 180.00 |

The process of granulate manufacturing included the following steps:
- pre-calibration / unification of starting materials (granulation components, including the APIs),
- mixing of the aforementioned components to obtain a uniform distribution of the active substance within the granulation bed (except composition 2). In the case of composition 2, in the first step, all components were mixed except the lubricating and anti-adhesive substance which was mixed in the second step,
- dry granulation using an Alexanderwerk BT 120 Pharma compactor, of the aforementioned mixture at standard process parameters, in order to obtain briquettes which were subsequently processed into fine particles and unified 1.2.3. Unprocessed Drotaverine and Caffeine added to the final tableting mixture in the form of loose powders, without the preliminary step of the treatment to obtain intermediates.

Both the powder comprising Drotaverine and the powder comprising Caffeine were prepared by mixing, respectively, Drotaverine and Caffeine with, for example, a filler, in order to avoid segregation.

### 2. Examples of pharmaceutical compositions according to the invention

Of the aforementioned intermediates of Metamizole, Drotaverine and Caffeine, examples of formulations were selected, which, in various combinations, were used to create compositions of one- and two-layered tablets. However, it should be clearly stressed that each combination of intermediates of metamizole/drotaverine/caffeine, processed to the form of a tablet, was characterised by a clearly higher stability than that of the reference product (Quarelin).

**Table 5. Preferred embodiments of combinations of intermediates of metamizole, drotaverine and caffeine in the final pharmaceutical composition according to the invention.**

| **Active substance** | **Type of intermediate** | | | | | | |
|---|---|---|---|---|---|---|---|
| MET | **Wet granulate of MET** | *Wet granulate of MET* | *Wet granulate of MET* | **Wet granulate of MET** | *Wet granulate of MET* | **Wet granulat*e* of MET** | 1L***:Wet granulate of MET** |
| DRO | DRO | DRO | *Dry granulate of DRO+CA F* | *Dry granulate of DRO+CA F* | *Dry granulat e of DRO* | *Dry granulate of DRO* | 2L*: *Dry granulate of DRO*+*CA F* |
| CAF | CAF | CAF | | | CAF | CAF | |
| DRO - Drotaverine hydrochloride, MET - Metamizole sodium, CAF - Caffeine, 1L/2L - bilayer tablet (1L, 2L - first layer, second layer, respectively) | | | | | | | |

The selected, exceptionally preferable intermediates, subsequently processed to the final form of a one-layered tablet or a two-layered tablet in one tableting step on a tableting machine, for example Korsch XL 100, or Kilian, optionally adding additional auxiliary substances for the tableting process, for example a lubricant such as, for example, magnesium stearate, talc, or a mixture thereof. Preferably, a disintegrant such as, for example, croscarmellose, may be added.

The tablets thus obtained were subsequently coated in order to mask the unpleasant flavour, using typical coatings prepared by dissolving in water the ready-to-use mixtures based on a film-forming polymer, e.g. HMPC or PVA (e.g. Opadry®), available on the market.

**Table 5. Preferred embodiments of pharmaceutical compositions according to the invention.**

| Composition No | **F001** | **F002** | F003 | F004 | F005 | F006 | **F007** |
|---|---|---|---|---|---|---|---|
| MET | MW3 | *MD2* | *MD5* | MW2 | *MD5* | MW1 | 1L: MW3 |
| DRO | DRO | DRO | *DC3* | *DC2* | *D4* | *D5* | 2L: *Dry granulate of DRO*+*CAF DC3* |
| CAF | CAF | CAF | | | CAF | CAF | |
| DRO - Drotaverine hydrochloride, MET - Metamizole sodium, CAF - Caffeine | | | | | | | |

The table below presents a list of qualitative and quantitative compositions as well as the manufacturing technologies for compositions F001, F002, F007 as well as for Quarelin. The presented qualitative and quantitative compositions of intermediates, included in the final compositions, were selected from the previously described intermediates. The subsequent table for the aforementioned compositions and Quarelin presents chemical stability results confirming the considerably better chemical stability of our compositions in relation to Quarelin. A description of the composition and of the manufacturing technology for each composition can also be found below.

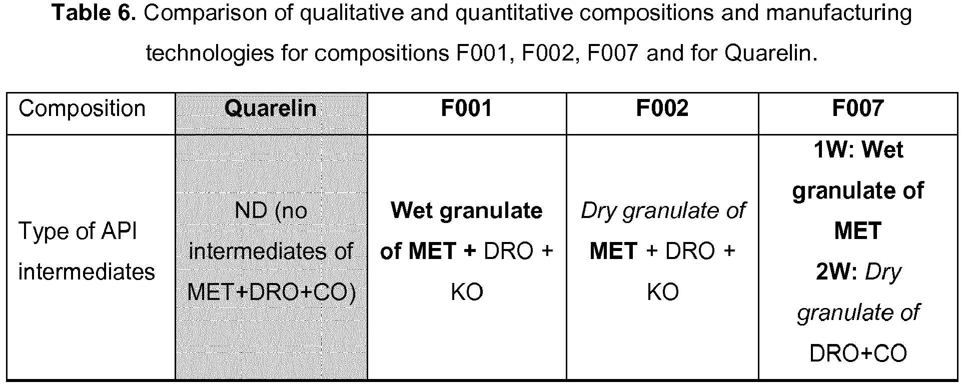

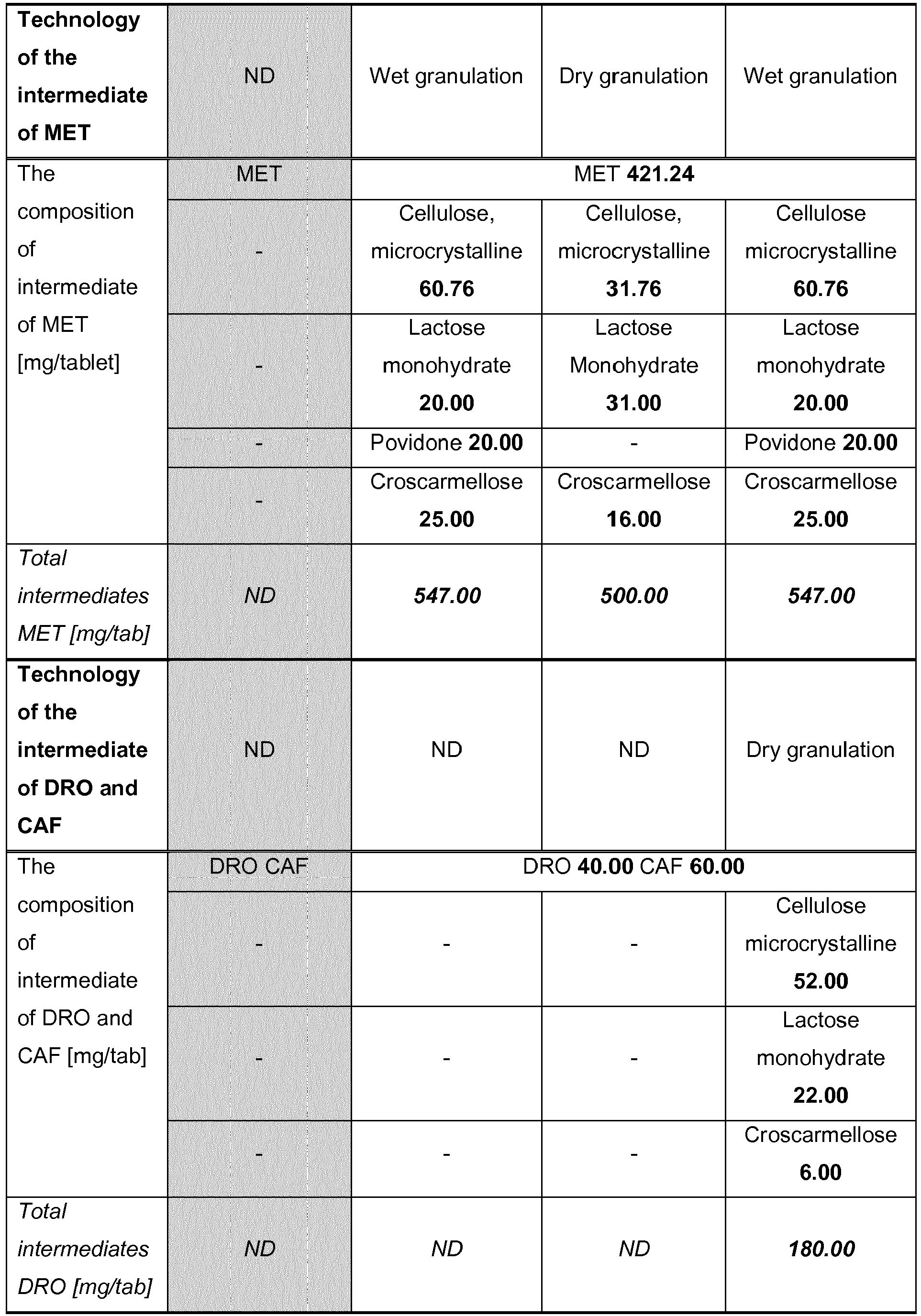

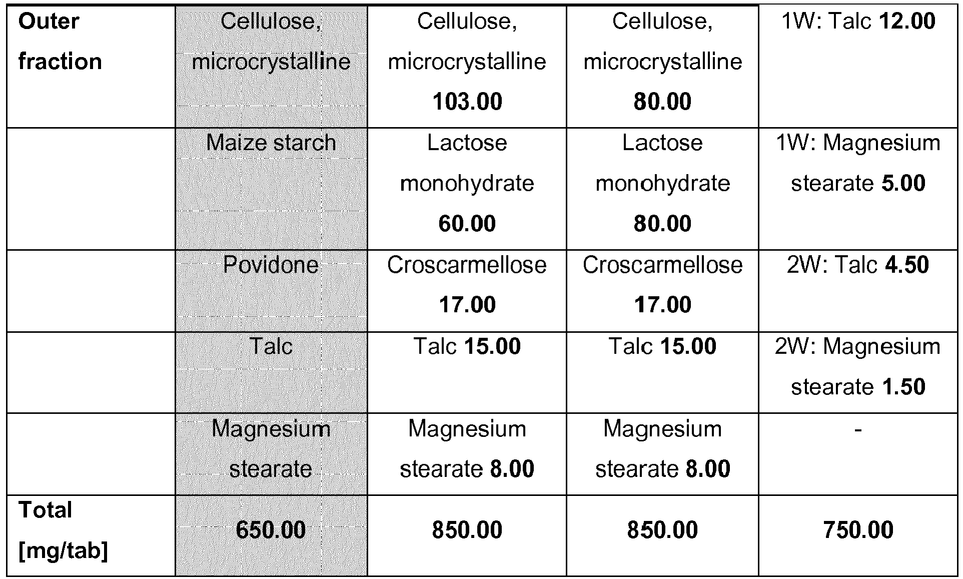

A detailed description of the manufacturing of examples of the compositions according to the invention

### Composition F001:

Has a qualitative composition provided in the above table. The outer fraction are the normally used auxiliary substances i.e. fillers (microcrystalline cellulose and lactose monohydrate), disintegrants (Croscarmellose), and lubricating substances (talc, magnesium stearate). The process of manufacturing a one-layered tablet involved the following actions:
1. Manufacturing of the granulate of MET by means of:
   - Pre-calibration / unification of starting materials (granulation bed components, including the APIs),
   - mixing of the aforementioned in a high-shear granulator to obtain a uniform distribution of the active substance within the granulation bed,
   - Preparation of an aqueous solution of the binder and the application thereof onto the bed,
   - Proper granulation (manufacturing of the granulate),
   - Drying of the granulate in order to remove water provided in the binder solution,
   - Calibration / unification of the dry granulate
2. Pre-calibration/unification of the outer fraction components, including DRO and CAF,
3. Preparation of Drotaverine premix by mixing with one with microcrystalline cellulose, in order to prevent segregation,
4. Preparation of Caffeine premix by mixing with lactose monohydrate, in order to prevent segregation,
5. Preparation of mixture I comprising the intermediate of MET, DRO premix, CAF premix, and the remaining substances except Magnesium Stearate,
6. Mixing of mixture I using a container mixer in order to obtain a uniform distribution of active substances within the mixture,
7. Mix the obtained mixture mentioned in point 6 with the lubricant (Magnesium Stearate)
8. Tablet the obtained tableting blend on a rotational tableting machine using punches with a desired size and shape.

### Composition F002:

Has a qualitative composition provided in the above table. Composition 002 differs from 001 in the technology of manufacturing of the intermediate of MET, which, in this composition, is obtained by means of dry granulation. Similarly to composition 001, Drotaverine and Caffeine were added without technological pre-treatment, within the outer fraction. The provided qualitative and quantitative composition for MET granulate is one of the compositions included in Table 2. The outer fraction are the normally used auxiliary substances i.e. fillers (microcrystalline cellulose and lactose monohydrate), disintegrants (Croscarmellose), and lubricating substances (talc and magnesium stearate). The process of manufacturing a one-layered tablet involved the following actions:
1. Manufacturing of the granulate of MET by means of:
   - Pre-calibration / unification of starting materials (granulation components, including the APIs),
   - mixing of the aforementioned components to obtain a uniform distribution of the active substance within the granulation bed,
   - Dry granulation of the aforementioned mixture at standard process parameters, in order to obtain briquettes which were subsequently processed into fine particles and unified.
2. Pre-calibration/unification of the outer fraction components, including DRO and CAF,
3. Preparation of Drotaverine premix by mixing with microcrystalline cellulose in order to prevent segregation,
4. Preparation of Caffeine premix by mixing with lactose monohydrate, in order to prevent segregation,
5. Preparation of mixture I comprising the intermediate of MET, DRO premix, CAF premix, and the remaining substances except Magnesium Stearate,
6. Mixing of mixture I using a container mixer in order to obtain a uniform distribution of active substances within the mixture,
7. Mix the obtained mixture mentioned in point 6 with the lubricant (Magnesium Stearate),
8. Tablet the obtained tableting blend on a rotational tableting machine using punches with a desired size and shape.

### Composition F007:

Composition 007 was developed with the intention of manufacturing a two-layered tablet which would enable the additional separation of the most incompatible APIs. The first layer comprises the intermediate of MET, the granulate manufactured using the wet granulation technology, and, additionally, lubricants. The second layer comprises the common granulate of Drotaverine and Caffeine, manufactured using the dry granulation technology. Both intermediates are compositions selected from the previously described intermediates (see Tables 1 and 3). The process of manufacturing the tablet involved the following steps:
1. Manufacturing of the granulate of MET by means of:
   - Pre-calibration / unification of starting materials (granulation bed components, including the APIs),
   - mixing of the aforementioned in a high-shear granulator to obtain a uniform distribution of the active substance within the granulation bed,
   - Preparation of an aqueous solution of the binder and the application thereof onto the bed,
   - Proper granulation (manufacturing of the granulate),
   - Drying of the granulate in order to remove water provided in the binder solution,
   - Calibration / unification of the dry granulate,
2. Manufacturing of the granulate of DRO+CAF by means of:
   - pre-calibration / unification of starting materials (granulation components, including the APIs),
   - mixing of the aforementioned components to obtain a uniform distribution of the active substance within the granulation bed,
   - dry granulation of the aforementioned mixture at standard process parameters, in order to obtain briquettes which were subsequently processed into fine particles and unified,
3. Preparation of a I layer mixture comprising MET granulate and talc (1st mixing step) and Magnesium Stearate (2nd mixing step),
4. Preparation of a II layer mixture comprising DRO+CAF granulate and talc (1st mixing step) and Magnesium Stearate (2nd mixing step),
5. Tableting of the aforementioned mixtures using a tableting machine that enables the manufacturing of two-layered tablets.

### 3. Testing for chemical stability of pharmaceutical compositions according to the invention

Stabilities of the examples of pharmaceutical compositions according to the invention were tested in accordance with current standards for chemical purity testing, using HPLC by means of analytical methods developed by own R&D Department.

From the data provided in Table 6 presenting the testing results, it follows that all compositions according to the invention exhibited, at the start, significantly better chemical purity than that of the product Quarelin available on the market. In addition, during the storage under conditions of 30°C/65%RH, these compositions were characterised by a much slower increase in impurities. The method for manufacturing the intermediate of MET affected the level of the main impurity of MET, but it did not increase over time and was significantly below the limit. Both the known impurities of Drotaverine (Drotaveraldine) and those unknown in the compositions described by us were at a much lower level than those in Quarelin. In summary, each of the compositions described by us guarantees obtaining better chemical stability than the preparation available on the market does.

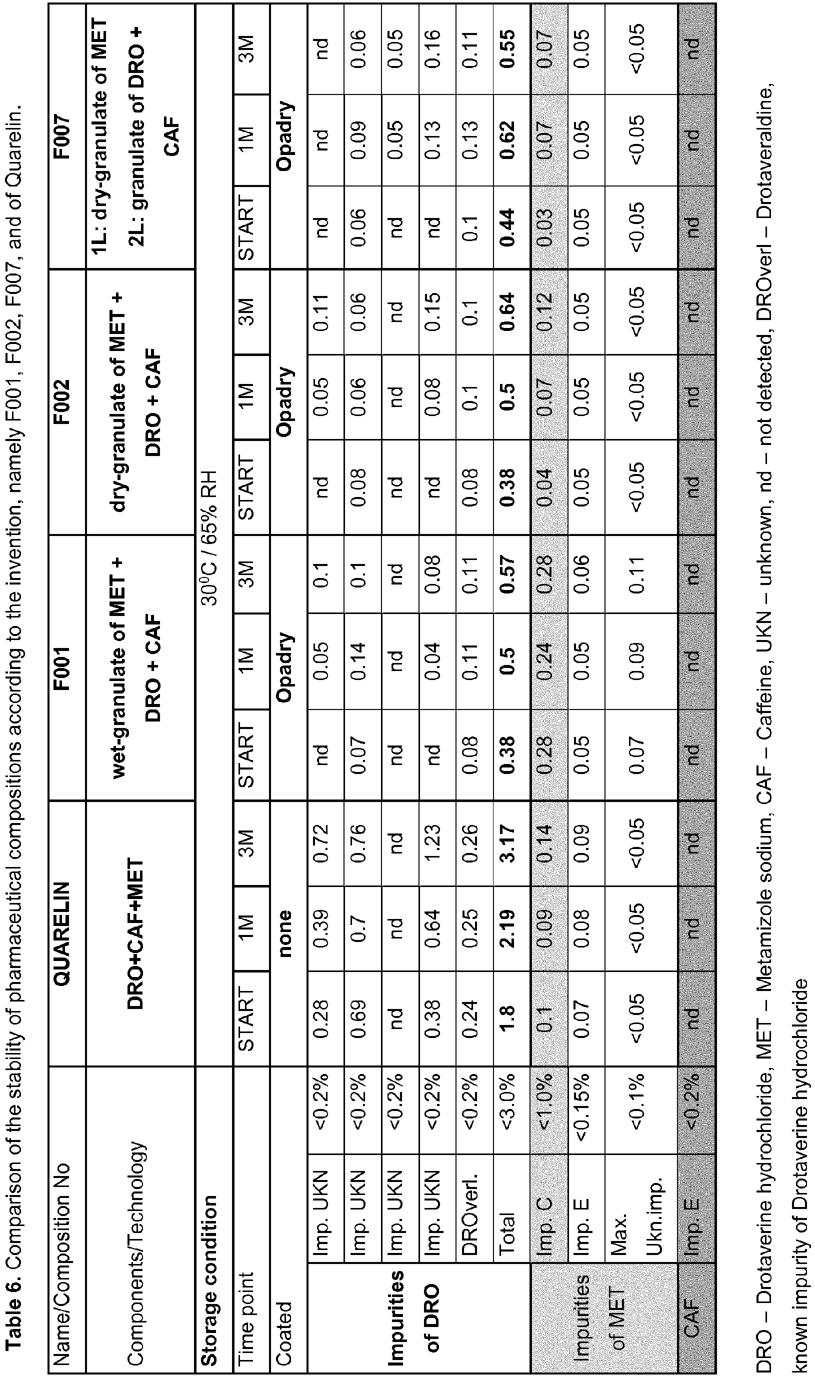

## Claims

1. A pharmaceutical composition comprising, in one dosage form, metamizole, drotaverine, and caffeine, **characterised in that** metamizole in the said composition is present in the form of a granulate comprising metamizole, and drotaverine and caffeine are present outside the said granulate.

2. A composition according to claim 1, **characterised in that** the granulate comprising metamizole is a wet granulate.

3. A composition according to claim 1, **characterised in that** the granulate comprising metamizole is a dry granulate.

4. A composition according to 2-3, **characterised in that** the granulate comprising metamizole consists of:
metamizole at an amount of 70 to 90% wt.,
at least one filler at an amount of 5 to 20% wt.,
at least one binder at an amount of 0 to 5% wt.,
at least one disintegrant at an amount of 0 to 5% wt.,
at least one lubricant at an amount of 0 to 1% wt.,
wherein the percentages are each time related to the total weight of the granulate, and each time sum up to 100%.

5. A composition according to 1-3, **characterised in that** drotaverine and caffeine are present together in one dry granulate comprising drotaverine and caffeine.

6. A composition according to 5, **characterised in that** the granulate comprising drotaverine and caffeine consists of:
drotaverine at an amount of 15 to 30% wt.,
caffeine at an amount of 25-35% wt.,
at least one filler at an amount of 30 to 50% wt.,
at least one binder at an amount of 0 to 5% wt.,
at least one disintegrant at an amount of 0 to 5% wt.,
at least one lubricant at an amount of 0 to 6% wt.,
wherein the percentages are each time related to the total weight of the granulate, and each time sum up to 100%.

7. A composition according to 1-3, **characterised in that** drotaverine is present in the form of a dry granulate comprising drotaverine, while caffeine is present in the form of a powder outside the said granulate comprising drotaverine.

8. A composition according to 7, **characterised in that** the granulate comprising drotaverine consists of:
drotaverine at an amount of 20 to 55% wt.,
at least one filler at an amount of 40 to 75% wt.,
at least one binder at an amount of 0 to 5% wt.,
at least one disintegrant at an amount of 0 to 5% wt.,
at least one lubricant at an amount of 0 to 6% wt.,
wherein the percentages are each time related to the total weight of the granulate, and each time sum up to 100%.

9. A composition according to 1-3, **characterised in that** drotaverine and caffeine are present in the form of a mixture of powders.

10. A composition according to claim 4 or 6 or 8, **characterised in that**
at least one filler is selected from a group consisting of lactose, microcrystalline cellulose, and maize starch,
at least one binder is selected from a group consisting of PVP, HPMC,
at least one disintegrant is selected from a group consisting of crospovidone, croscarmellose
at least one lubricant is selected from a group consisting of talc and magnesium stearate.

11. A pharmaceutical composition according to any of the above claims, **characterised in that** metamizole is in the form of metamizole sodium monohydrate, drotaverine is in the form of drotaverine hydrochloride, and caffeine is in the form of caffeine free base

12. A dry granulate comprising drotaverine, **characterised in that** it comprises no acid stabilisers.

13. A granulate according to claim 12, **characterised in that** it consists of:
drotaverine at an amount of 20 to 55% wt.,
at least one filler at an amount of 40 to 75% wt.,
at least one binder at an amount of 0 to 5% wt.,
at least one disintegrant at an amount of 0 to 5% wt.,
at least one lubricant at an amount of 0 to 6% wt.,

14. A granulate according to claim 13, **characterised in that** it consists of:
at least one filler is selected from a group consisting of lactose, microcrystalline cellulose, and maize starch,
at least one binder is selected from a group consisting of PVP, HPMC,
at least one disintegrant is selected from a group consisting of crospovidone, croscarmellose
at least one lubricant is selected from a group consisting of talc and magnesium stearate.

15. The use of the granulate as per any of claims 12-14 for the manufacturing of a pharmaceutical composition comprising drotaverine.

16. The use of the granulate comprising metamizole for the manufacturing of a pharmaceutical composition comprising metamizole, drotaverine, and caffeine.
